(19) Europäisches Patentamt
European Patent Office
Office européen des brevets

(11) Publication number: 0 366 287 A1

(12) # EUROPEAN PATENT APPLICATION

(21) Application number: 89310146.9

(51) Int. Cl.[5]: A61K 31/70

(22) Date of filing: 04.10.89

(30) Priority: 05.10.88 GB 8823353

(43) Date of publication of application:
02.05.90 Bulletin 90/18

(84) Designated Contracting States:
AT BE CH DE FR GB IT LI LU NL SE

(71) Applicant: THE WELLCOME FOUNDATION LIMITED
183-193 Euston Road
London NW1 2BP(GB)

(72) Inventor: Koszalka, George Walter
117 Priestly Creek Drive
Chapel Hill North Carolina 27514(US)
Inventor: Averett, Devron Randolph
2608 Lochmore Drive
Raleigh North Carolina 27608(US)
Inventor: Burns, Charlene Louise
1418 North Duke Street
Durham North Carolina 27701(US)
Inventor: Krenitsky, Thomas Anthony
106 Laurel Hill Road
Chapel Hill North Carolina 27514(US)

(74) Representative: Garrett, Michael et al
The Wellcome Research Laboratories Group
Patents and Agreements Langley Court
Beckenham Kent BR3 3BS(GB)

(54) **Therapeutic nucleosides.**

(57) 2′,3′-Dideoxypurine nucleosides and pharmaceutically acceptable derivatives thereof in combination with zidovudine; pharmaceutical formulations containing said combinations; their use in medical therapy, particularly in the treatment or prophylaxis of viral infections.

EP 0 366 287 A1

## Therapeutic Nucleosides

The present invention relates to certain 2′,3′-dideoxypurine nucleosides and pharmaceutically acceptable derivatives thereof in combination with zidovudine, pharmaceutical formulations containing said combinations and their use in medical therapy, particularly for the treatment or prophylaxis of viral infections.

AIDS is an immunosuppressive or immunodestructive disease that predisposes subjects to fatal opportunistic infections. Characteristically, AIDS is associated with a progressive depletion of T-cells, especially the helper-inducer subset bearing the OKT4 surface marker.

Human immunodeficiency virus (HIV) has been reproducibly isolated from patients with AIDS or with the symptoms that frequently precede AIDS. HIV is cytopathic and appears to preferentially infect and destroy T-cells bearing the OKT4 marker, and it is now generally recognized that HIV is the etiological agent of AIDS. Infection with HIV is also associated with clinical conditions such as AIDS-related complex (ARC), progressive generalised lymphadenopathy (PGL), AIDS-related neurological conditions, such as multiple sclerosis or tropical paraparesis, anti-HIV antibody-positive and HIV-positive conditions, Kaposi's sarcoma and thrombocytopenic purpura.

Since the discovery that HIV is the etiological agent of AIDS, numerous proposals have been made for anti-HIV chemotherapeutic agents that may be effective in treating AIDS sufferers. Thus, for example, European Patent Specification No. 196185 describes 3′-azido-3′-deoxythymidine (which has the approved name zidovudine), its pharmaceutically acceptable derivatives and their use in the treatment of human retrovirus infections including AIDS and associated clinical conditions.

European Patent Publication No. 0206497 relates generally to 2′,3′-dideoxy purine nucleosides and their use in the treatment of HIV infections and related conditions. Other 2′,3′-dideoxypurine nucleosides have previously been described, in particular 6-methylaminopurine-9-β-D-2′-,3′-dideoxyribo furanoside has been disclosed in Bioorg Khim 9(1) 52-59 (1983). European Patent Publication No. 0286425 discloses 6-substituted 2′,3′-dideoxynucleosides.

Another group of viral pathogens of major consequence worldwide are the hepatitis viruses, in particular hepatitis B virus (HBV). Clinical affects of infection with HBV range from headache, fever, malaise, nausea, vomiting, anorexia and abdominal pains. Replication of the virus is usually controlled by the immune response, with a cause of recovery lasting weeks or months in humans, but infection may be more severe leading to persistent chronic liver disease. In "Viral Infections of Humans" (Second Edition, Ed., Evans, A.S. (1982) Plenum Publishing Corporation, New York), Chapeter 12 describes in detail the etiology of viral hepatitis infections.

We have now discovered that zidovudine in combination with certain 2′,3′-dideoxypurine nucleosides results in a large potentiation of the anti-HIV activity of the compounds.

According to a first feature of the present invention there is provided a combination of (a) zidovudine and (b) at least one 2′,3′-dideoxypurine nucleoside selected from:

i) 6-Ethylaminopurine-9-β-D-2′,3′-dideoxyribofuranoside;
ii) 6-Dimethylaminopurine-9-β-D-2′,3′-dideoxyribofuranoside;
iii) 6-Cyclopropylaminopurine-9-β-D-2′,3′-dideoxyribofuranoside;
iv) 6-n-Propoxypurine-9-β-D-2′,3′-dideoxyribofuranoside;
v) 6-Cyclopropylmethoxypurine-9-β-D-2′,3′-dideoxyribofuranoside;
vi) 6-Cyclopentyloxypurine-9-β-D-2′,3′-dideoxyribofuranoside,
vii) 2-Amino-6-ethoxypurine-9-β-D-2′,3′-dideoxyribofuranoside;
viii) 6-sec-Butoxypurine-9-β-D-2′,3′-dideoxyribofuranoside; and
ix) 6-[Cyclopropylmethylamino]purine-9-β-D-2′,3′-dideoxyribofuranoside. or a pharmaceutically acceptable derivative thereof, whereby components (a) and (b) of the combination being employed together such that a potentiated antiviral effect is achieved.

The term a "potentiated antiviral effect" is used herein to denote an antiviral effect for the above combination of components a) and b) which is greater than the sum of the antiviral effects of the individual components.

Most preferred combinations are those wherein component (b) is iii) or ix) above.

It should be noted that the invention includes all isomeric and tautomeric forms of the components (a) and (b) of the combination, either alone or in admixture.

Examples of viral infections and associated clinical conditions which may be treated or prevented in accordance with the invention include human retroviral infections such as human immunodeficiency virus (HIV), e.g. HIV-I or HIV-II, or human T-cell lymphotropic virus (HLTV) e.g. HTLV-I or HTLV-II infections. The

combinations of the present invention are especially useful for the treatment or prophylaxis of AIDS and related clinical conditions such as AIDS-related complex (ARC), progressive generalized lymphadenopathy (PGL), AIDS-related neurological conditions, such as multiple sclerosis or tropical paraparesis, anti-HIV antibody-positive patients, and HIV-positive conditions such as Kaposi's sarcoma and thrombocytopenic purpura. The combinations of the present invention may also be used in the treatment or prevention of psoriasis.

The combinations of the present invention have been found to be particularly applicable to the treatment of asymptomatic infections or diseases in humans caused by or associated with human retroviruses.

The combinations of the present invention may also be used in the treatment or prophylaxis of hepatitis B virus infections and associated clinical disorders.

In a second aspect of the present invention there is provided a 2′,3′-dideoxypurine nucleoside as hereinbefore described or a pharmaceutically acceptable derivative thereof for use in combination therapy with zidovudine. In particular, there is provided a 2′,3′-dideoxypurine nucleoside as herein before described or a pharmaceutically acceptable derivative thereof, for use in combination therapy with zidovudine, whereby a potentiated antiviral effect is achieved.

It will be appreciated that, while there will usually be an optimum ratio of the components to ensure maximum potentiation, even a vanishingly small quantity of one component will suffice to potentiate the effect of the other to some degree, and so any ratio of two potentiating components will still possess the required synergistic effect. However, greatest synergy is generally observed when the two components are present in particular parts.

Thus, the optimum ratio range, in µmoles, of zidovudine to a 2′-, 3′-dideoxypurine nucleoside described in the present invention is 10:1 to 1:100, preferably 1:1 to 1:75, more preferably 1:1 to 1:40, and most preferably 1:1 to 1:20.

The combinations described above are referred to hereafter as "combinations according to the invention" or "active ingredient(s)".

Thus, there is further provided:

i) a combination according to the invention for use in medical therapy particularly in the treatment or prophylaxis of any of the infections or conditions hereinbefore mentioned,

ii) use of a combination according to the invention in the manufacture of a medicament for the treatment or prophylaxis of any of the infections or conditions hereinbefore mentioned;

iii) a method for the treatment of viral infections in a human or animal body which comprises administering to said human or animal body an effective amount of a combination according to the invention.

The present invention further includes a process for preparing the combinations according to the invention which comprises bringing into association a 2′,3′-dideoxypurine nucleoside as hereinbefore described and zidovudine, in a medicament to provide a potentiated antiviral effect.

By "a pharmaceutically acceptable derivative" is meant any pharmaceutically acceptable salt, ester, or salt of such ester, of a 2′,3′-dideoxypurine nucleoside or any other compound which, upon administration to a human or animal (the "recipient"), is capable of providing (directly or indirectly) the parent 2′,3′-dideoxypurine nucleoside or an antivirally active metabolite or residue thereof.

Preferred esters of the 2′,3′-dideoxypurine nucleoside include carboxylic acid esters in which the non-carbonyl moiety of the ester grouping is selected from straight or branched chain alkyl e.g. n-propyl, t-butyl, n-butyl, alkoxyalkyl (e.g. methoxymethyl), aralkyl (e.g. benzyl), aryloxyalkyl (e.g. phenoxymethyl), aryl (e.g. phenyl optionally substituted by halogen, $C_{1-4}$ alkyl or $C_{1-4}$ alkoxy); sulphonate esters such as alkyl- or aralkylsulphonyl (e.g. methanesulphonyl), amino acid esters (e.g. L-valyl or L-isoleucyl); and mono-, di- or tri-phosphate esters.

With regard to the above-described esters, unless otherwise specified, any alkyl moiety present advantageously contains 1 to 18 carbon atoms, particularly 1 to 4 carbon atoms. Any aryl moiety present in such esters advantageously comprises a phenyl group.

Examples of pharmaceutically acceptable salts of the 2′,3′-dideoxypurine nucleoside include base salts, eg derived from an appropriate base, such as alkali metal (e.g. sodium), alkaline earth metal (e.g. magnesium) salts, ammonium and $NX_4^+$ (wherein X is $C_{1-4}$ alkyl). Pharmaceutically acceptable salts of a hydrogen atom or an amino group include salts of organic carboxylic acids such as acetic, lactic, tartaric, malic, isethionic, lactobionic and succinic acids; organic sulfonic acids such as methanesulfonic, ethanesulfonic, benzenesulfonic and p-toluenesulfonic acids and inorganic acids such as hydrochloric, sulfuric, phosphoric and sulfamic acids. Pharmaceutically acceptable salts of a compound with a hydroxy group include the anion of said compound in combination with a suitable cation such as $Na^+$, $NH_4^+$ and $NX_4^+$ -

(wherein X is a $C_{1-4}$ alkyl group).

An advantage of the combination of the present invention is that it enables attainment of an improved antiviral efficacy at a particular dose of one of the antiviral components (compared with the component used alone) thereby improving the therapeutic index of the component. Thus, for example, the combination may be used to treat conditions which would otherwise require relatively large doses of the antiviral component at which toxicity problems may occur. The smaller doses of the combination may provide increased convenience to the patient and increased compliance.

The components a) and b) of the combinations according to the invention may conveniently be administered together, for example, in a unitary pharmaceutical formulation, or separately, for example as a combination of different pharmaceutical formulations e.g. tablets and injections, administered simultaneously or sequentially . In the latter case, however the components, are administered within a sufficiently short interval to ensure that a potentiated antiviral effect is achieved.

The combinations according to the invention may be administered by any route appropriate to the condition to be treated, suitable routes including oral, rectal, nasal, topical (including buccal and sublingual), vaginal and parenteral (including subcutaneous, intramuscular, intravenous, intradermal, intrathecal and epidural). It will be appreciated that the preferred route may vary with, for example, the condition of the recipient.

For the treatment of any of the clinical conditions associated with viral infections as outlined above the amount required of the individual active ingredient will depend upon a number of factors including the severity of the condition to be treated and the identity of the recipient and will ultimately be at the discretion of the attendant physician. In general, however, for each of the conditions, a suitable, effective dose of a 2′,3′-dideoxypurine nucleoside will be in the range 1-300 mg per kilogram body weight of recipient per day, preferably in the range 5-250 mg per kilogram body weight per day and most preferably in the range 10-200 mg per kilogram body weight per day, a typical daily dose might be 1-40 mg per kilogram body weight per day. (unless otherwise indicated all weights of the 2′,3′-dideoxypurine nucleoside are calculated as the parent compound; for salts and esters thereof the figures would be increased proportionately.) The dose may be presented as two, three, four or more sub-doses administered at appropriate intervals throughout the day. These sub-doses may be administered in unit dosage forms, for example, containing 10 to 1000 mg, preferably 20 to 500 mg and most preferably 100 to 400 mg of the 2′,3′-dideoxypurine nucleoside per unit dosage form.

In general a suitable dose of zidovudine will be in the range of 0.5-40 mg per kilogram body weight of the recipient per day, preferably in the range of 2-30 mg per kilogram body weight per day and most preferably in the range of 4-20 mg per kilogram body weight per day. The desired dose is preferably presented as two, three, four, five, six or more sub-doses administered at appropriate intervals throughout the day. These sub-doses may be administered in unit dosage forms, for example, containing 10 to 1500 mg, preferably 20 to 1000 mg, and most preferably 50 to 700 mg of zidovudine per unit dosage form.

While it is possible for the combinations according to the invention to be administered alone, it is preferable to present them as pharmaceutical formulations. The formulations of the present invention comprise a combination, as above defined, together with one or more acceptable carriers thereof and optionally other therapeutic ingredients. The carrier(s) must be "acceptable" in the sense of being compatible with the other ingredients of the formulation and not deleterious to the recipients thereof.

The formulations include those suitable for oral, rectal, nasal, topical (including buccal and sublingual), vaginal or parenteral (including subcutaneous, intramuscular, intravenous, intradermal, intrathecal and epidural) administration. The formulations may conveniently be presented in unit dosage form and may be prepared by any of the methods well known in the art of pharmacy. Such methods include the step of bringing into association the active ingredient with the carrier which constitutes one or more accessory ingredients. In general the formulations are prepared by uniformly and intimately bringing into association the active ingredient with liquid carriers or finely divided solid carriers or both, and then, if necessary, shaping the product.

Formulations of the present invention suitable for oral administration may be presented as discrete units such as capsules, cachets or tablets each containing a predetermined amount of the active ingredient; as a powder or granules; as a solution or a suspension in an aqueous liquid or a non-aqueous liquid; or as an oil-in-water liquid emulsion or a water-in-oil liquid emulsion. The active ingredient may also be presented as a bolus, electuary or paste.

A tablet may be made by compression or moulding, optionally with one or more accessory ingredients. Compressed tablets may be prepared by compressing in a suitable machine the active ingredient in a free-flowing form such as a powder or granules, optionally mixed with a binder (e.g. povidone, gelatin, hydroxypropylmethyl cellulose), lubricant, inert diluent, disintegrant (e.g. sodium starch glycollate, cross-linked povidone, cross-linked sodium carboxymethyl cellulose), surface-active or dispersing agent. Moulded tablets may be made by moulding in a suitable machine a mixture of the powdered compound moistened with an inert liquid diluent. The tablets may optionally be coated or scored and may be formulated so as to provide slow or controlled release of the active ingredient therein using, for example, hydroxypropylmethylcellulose in varying proportions to provide desired release profile. Tablets may optionally be provided with an enteric coating, to provide release in parts of the gut other than the stomach. This is particularly advantageous for purine nucleoside derivatives as such compounds are susceptible to acid hydrolysis.

Formulations suitable for oral use as described above may also include buffering agents designed to neutralise stomach acidity. Such buffers may be chosen from a variety of organic or inorganic agents such as weak acids or bases admixed with their conjugated salts.

For infections of the eye or other external tissues, e.g., mouth and skin, the formulations are preferably applied as a topical ointment or cream containing the active ingredient in an amount of, for example, 0.075 to 20% w/w, preferably 0.2 to 15% w/w and most preferably 0.5 to 10% w/w. When formulated in an ointment, the active ingredients may be employed with either a paraffinic or a water-miscible ointment base. Alternatively, the active ingredients may be formulated in a cream with an oil-in-water cream base or as a water-in-oil base. Topical administration may be by means of a transdermal iontophoretic device.

If desired, the aqueous phase of the cream base may include, for example, at least 40-45% w/w of a polyhydric alcohol, i.e. an alcohol having two or more hydroxyl groups such as propylene glycol, butane-1,3-diol, mannitol, sorbitol, glycerol and polyethylene glycol and mixtures thereof. The topical formulations may desirably include a compound which enhances absorption or penetration of the active ingredient through the skin or other affected areas. Examples of such dermal penetration enhancers include dimethylsulphoxide and related analogues.

The oily phase of an emulsion formulation according to the invention may comprise merely an emulsifier (otherwise known as an emulgent), but desirably comprises a mixture of at least one emulsifier with a fat or an oil or with both a fat and an oil. Preferably, a hydrophilic emulsifier is included together with a lipophilic emulsifier which acts as a stabilizer. It is also preferred to include both an oil and a fat. Together, the emulsifer(s) with or without stabilizer(s) make up the so-called emulsifying wax, and the wax together with the oil and/or fat make up the so-called emulsifying ointment base which forms the oily dispersed phase of the cream formulations.

Emulgents and emulsion stabilizers suitable for use in the formulation of the present invention include Tween 60, Span 80, cetostearyl alcohol, myristyl alcohol, glyceryl mono-stearate and sodium lauryl sulphate.

The choice of suitable oils or fats for the formulation is based on achieving the desired cosmetic properties, since the solubility of the active compound in most oils likely to be used in pharmaceutical emulsion formulations is very low. Thus the cream should preferably be a non-greasy, non-staining and washable product with suitable consistency to avoid leakage from tubes or other containers. Straight or branched chain, mono- or dibasic alkyl esters such as di-isoadipate, isocetyl stearate, propylene glycol diester of coconut fatty acids, isopropyl myristate, decyl oleate, isopropyl palmitate, butyl stearate, 2-ethylhexyl palmitate or a blend of branched chain esters known as Crodamol CAP may be used, the last three being preferred esters. These may be used alone or in combination depending on the properties required. Alternatively, high melting point lipids such as white soft paraffin and/or liquid paraffin or other mineral oils can be used.

Formulations suitable for topical administration to the eye also include eye drops wherein the active ingredient is dissolved or suspended in a suitable carrier, especially an aqueous solvent for the active ingredient. The active ingredient is preferably present in such formulations in a concentration of 0.5 to 20%, advantageously 0.5 to 10% particularly about 1.5% w/w.

Formulations suitable for topical administration in the mouth include lozenges comprising the active ingredient in a flavoured basis, usually sucrose and acacia or tragacanth; pastilles comprising the active ingredient in an inert basis such as gelatin and glycerin, or sucrose and acacia; and mouth-washes comprising the active ingredient in a suitable liquid carrier.

Formulations for rectal administration may be presented as a suppository with a suitable base comprising for example cocoa butter or higher fatty alcohol (e.g. hard wax, European Pharmacopoeia) or triglycerides and saturated fatty acids (e.g. Witepsol).

Formulations suitable for nasal administration wherein the carrier is a solid include a coarse powder having a particle size for example in the range 20 to 500 microns which is administered in the manner in which snuff is taken, i.e. by rapid inhalation through the nasal passage from a container of the powder held close up to the nose. Suitable formulations wherein the carrier is a liquid, for administration as for example a nasal spray or as nasal drops, include aqueous or oily solutions of the active ingredient.

Formulations suitable for vaginal administration may be presented as pessaries, tampons, creams, gels, pastes, foams or spray formulations containing in addition to the active ingredient such carriers as are known in the art to be appropriate.

Formulations suitable for parenteral administration include aqueous and non-aqueous sterile injection solutions which may contain anti-oxidants, buffers, bacteriostats and solutes which render the formulation isotonic with the blood of the intended recipient; and aqueous and non-aqueous sterile suspensions which may include suspending agents and thickening agents, and liposomes or other microparticulate systems which are designed to target the compound to blood components or one or more organs. The formulations may be presented in unit-dose or multi-dose containers, for example sealed ampoules and vials, and may be stored in a freeze-dried (lyophilized) condition requiring only the addition of the sterile liquid carrier, for example water for injections, immediately prior to use. Extemporaneous injection solutions and suspensions may be prepared from sterile powders, granules and tablets of the kind previously described.

Preferred unit dosage formulations are those containing a daily dose or unit, daily sub-dose, as herein above recited, or an appropriate fraction thereof, of an active ingredient.

It should be understood that in addition to the ingredients particularly mentioned above the formulations of this invention may include other agents conventional in the art having regard to the type of formulation in question, for example those suitable for oral administration may include flavouring agents.

The present invention further includes a process for the preparation of a combination according to the invention and pharmaceutically acceptable derivatives thereof which comprises (1) preparing a 2′,3′-dideoxypurine nucleoside by either:

(A) reacting a compound of formula I:

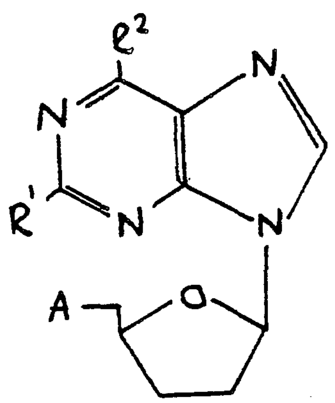

(I)

(wherein $R_1$ is H and; $R_2$ is selected from ethylamino, dimethylamino, cyclopropylamino, n-propoxy, cyclopropylmethoxy, cyclopentyloxy, sec-butoxy, cyclopropylmethylamino; or $R^1$ is $NH_2$ and $R^2$ is ethoxy; and A represents a precursor group for the hydroxy group; or a pharmaceutically acceptable derivative thereof) with an agent or under conditions serving to convert the said precursor group into the corresponding desired group; or

(B) reacting a purine base of formula II:

B - H (II)

(wherein B is the required purine moiety of a purine compound according to the invention; or a functional equivalent thereof, with a compound serving to introduce the desired dideoxy ribofuranosyl ring at the 9-position of the purine base of formula II; and thereafter, or simultaneously therewith, effecting one or more of the following optional conversions:-

(i) when a 2′,3′-dideoxypurine nucleoside is formed, converting it into a pharmaceutically acceptable derivative thereof,

(ii) when a pharmaceutically acceptable derivative of a 2′,3′-dideoxypurine nucleoside is formed, converting the said derivative into a 2′,3′-dideoxypurine nucleoside or a different derivative thereof, and (2) combining said 2′,3′-dideoxypurine nucleoside with zidovudine.

In the above-described process according to the invention, it will be appreciated that the precursor compounds of the 2′,3′-dideoxypurine nucleoside as well as the above-mentioned agents and conditions, will be selected from those that are known in the art of nucleoside synthetic chemistry. Examples of such conversion procedures are described hereinafter for guidance and it will be understood that they can be modified in conventional manner depending on the desired 2′,3′-dideoxypurine nucleoside. In particular, where a conversion is described which would otherwise result in the undesired reaction of labile groups

then such groups may be protected in conventional manner, with subsequent removal of the protecting groups after completion of the conversion.

With regard to process (A), A may represent a protected hydroxy group e.g. an ester grouping of the type referred to above particularly acetoxy, or an ether group such as a trialkylsilyloxy group, e.g. t-butyldimethylsilyloxy or an aralkoxy group e.g. triphenylmethoxy. Such groups may be converted for example by hydrolysis to the desired hydroxy group or, by transesterification, to an alternative ester group.

With regard to process (B), this may be effected for example by treating an appropriate purine base of formula (II) or a salt or protected derivative thereof, with a donor nucleoside capable of providing a 2′,3′-dideoxyribose sugar ring for example 3′-deoxythymidine preferably in the presence of an appropriate pentosyl transferring enzyme.

A 2′,3′-dideoxypurine nucleoside may be converted into a pharmaceutically acceptable phosphate or other ester by reaction with respectively a phosphorylating agent, e.g. $POCl_3$ or an appropriate esterifying agent, e.g. an acid halide or anhydride. The 2′,3′-dideoxypurine nucleoside, including esters thereof, may be converted into pharmaceutically acceptable salts thereof in conventional manner, e.g. by treatment with an appropriate base. An ester or salt of a 2′,3′-dideoxypurine nucleoside may be converted into the parent compound, e.g. by hydrolysis.

The following Examples are intended for illustration only and are not intended to limit the scope of the invention in any way.

Zidovudine is synthesised by the method described in EP Patent Specification No. 196185. The 2′,3′-dideoxy purine nucleosides may be prepared by the following examples.

## Example 1 : 6-Ethylaminopurine-9-β-D-2′,3′-dideoxyribofuranoside

6-Ethylaminopurine (prepared by nucleophilic displacement of the chlorine group on 6-chloropurine (Sigma Chemicals, St. Louis Mo) by the amino group of ethylamine) (2.69mmol, 0.5g) and 3′-deoxythymidine (Horwitz, J.P. et al, J.Org.Chem., 31 205 (1966)) (3.33mmol, 0.755g) were combined along with 50ml of 10mM potassium phosphate buffer with a pH of 6.8, containing 0.04% potassium azide. Purified thymidine phosphorylase (400 I. U.) and purine nucleoside phosphorylase (700 I. U.) were added and the suspension was stirred at 37°C. After 48 hours an additional 700 units of purine nucleoside phosphorylase and 400 units of thymidine phosphorylase were added, and the reaction was stirred at 37°C. Five days later the reaction was filtered, and the filtrate was applied to a column containing AG-1 X2 hydroxide resin (2.5 x 10cm). The product was eluted with a water wash and chromatographed on Amberlite XAD-2 resin (2.5 x 20cm). After sample application, this column was washed with a large volume of water. The product was eluted with methanol. After removal of the solvent, the product was redissolved in water and acetone then lyophilized yielding 6-ethylaminopurine-9-β-D-2′,3′-dideoxy ribofuranoside that analyzed for 0.2 water and 0.1 acetone (mp = < 30°C, [α]20°C = -29.45°C (0.5, DMF)).

$^1$H-NMR(200 MHz, DMSO-$d_6$) : δ 8.32(s, 1H,$H_8$), 8.17(s, 1H, $H_2$), 7.76(b 2H, $NH_2$), 6.20(dd, 1H, $H_1$), 6.20(t, 1H J = 5.6, 5′ OH), 4.09(m,1H, $H_4$), 3.53 (m, 4H, 5′, 5″$CH_2$ and $Ch_2$), 2.35(m, 2H, 2′ and 2″$CH_2$), 2.05(m, 2H, 3′ and 3″ $CH_2$), 1.14(t, 3H, J=7.03, $CH_3$)

Anal. Calcd. for $C_{12}H_{17}N_5O_2$ 0.2 $H_2O$ 0.1 $C_3H_6O$:
Calcd.: C, 54.17; H, 6.64; N, 25.68
Found: C, 54.13; H, 6.69, N, 25.75

## Example 2 : 6-Dimethylaminopurine-9-β-D-2′3′-dideoxyribofuranoside

6-Dimethylaminopurine (6.13 mmoles, 1g, Sigma Chemical Co., St. Louis, MO) and 3′-deoxythymidine (4.44 mmoles, 1g) (Horwitz, J. P. et al., J. Org. Chem., 31, 205 (1966) were suspended in 50ml of a 10 mM potassium phosphate solution pH 7.0 and containing 0.04% potassium azide. Purified thymidine phosphorylase (2000 I. U) and purine nucleoside phosphorylase (3000 I. U.) (Krenitsky T.A. et al., Biochemistry, 20, 3615, (1981) and US Patent 4,381,444) were added and the suspension stirred at 35°C. After 120 hours the reaction was filtered and the filtrate chromatographed on a column containing Dowex-1-hydroxide resin (2.5 x 8 cm) with 90% methanol and water (v/v) as the eluent. Fractions containing product were combined and the solvent removed under vacuum. The residue was dissolved in 25 ml 30% n-propanol/water (v/v) and chromatographed on a column containing BioRad P-2 (5 x 90cm). The product was eluted with 30% n-propanol/water (v/v). Product containing fractions were combined and the solvent removed under vacuum. The residue was dissolved in 30ml de-ionized water and chromatographed on a

column containing Sephadex G-10 resin (5 x 90cm). The eluent was water. Appropriate fractions were combined and after lyophilization yielded 6-dimethylaminopurine-9-β-D-2′,3′-dideoxyribofuranoside that analyzed as a 0.3 hydrate and (mp = 162° C).
$^1$H NMR (200 MHz, DMSO-$d_6$) : δ 8.38 (s, 1H, $H_8$), 8.21 (s, 1H, $H_2$), 6.25 (dd, 1H, $H_1$′), 5.07 (b, 1H, OH), 4.12 (m, 1H, $H_4$′), 3.65-3.40 (m, 2H, 5′$CH_2$), 3.35-3.6 (b, 6H, $CH_3$), 2.38 (m, 2H, 2′, $CH_2$), 2.05 (m, 2H, 3′ $CH_2$)
Anal. Calcd. for $C_{12}H_{17}N_5O_2 0.3H_2O$ C, 53.64; H, 6.60; N 26.06
Found: C, 53.63; H, 6.63; N, 25.8

Example 3 : 6-Cyclopropylaminopurine-9-β-D-2′,3′-dideoxyribofuranoside

6-Cyclopropylaminopurine (prepared from 6-chloropurine (Sigma Chemicals, St. Louis MO) and cyclopropylamine) (2.86 mmoles, 0.5 g) and 3′-deoxythymidine (4.30 mmoles, 1 g) (Horwitz J.P. et al J.Org.Chem., 31, 205 (1966)) were dissolved in 10 ml of a 1:1 dimethylsulfoxide: N′,N′-dimethylformamide mixture and further diluted with 30 ml of a 10 mM potassium phosphate buffer pH 6.8 and containing 0.04% potassium azide. Purified thymidine phosphorylase (10,000 I.U.) and purine nucleoside phosphorylase (20,000 I.U.) (Krenitsky T.A., et al, Biochemistry, 20, 3615, 1981 and US Patent 4,381,444) absorbed onto 10 ml of DEAE resin (Whatman) were added and the suspension was stirred at 35° C. After 8 hours the reaction was filtered and the filtrate was applied to a series of coupled columns. The initial column contained Dowex-1-hydroxide (2.5 x 10 cm) while the second column was filled with Amberlite XAD-2 resin (2.5 x 20 cm). After sample application, the columns were washed with a large volume of water and the product was eluted with methanol. Product containing fractions were combined and after lyophilization yielded 6-cyclopropylaminopurine-9-β-D-2′,3′-dideoxyribofuranoside that analyzed as a 0.55 hydrate and (mp = 63-65° C).
$^1$H NMR (200 MHz, DMSO-$d_6$) : δ 8.33 (s, 1H, $H_8$), 8.21 (s, 1H, $H_2$), 7.90 (d, 1H, J=3.76 Hz, NH), 6.21 (dd, 1H, $H_1$′), 5.03 (t, 1H, J=5.66 Hz, 5′OH), 4.08 (m 1H, $H_4$′), 3.53 (m, 2H, 5′$CH_2$), 3.03 (b, 1H, CH), 2.41 (m, 2H, 2′$CH_2$), 2.03 (m, 2H, 3′$CH_2$) and 0.70 and 0.58 (2 multiplets, 2H and 2H, 2-$CH_2$-)
Anal. Calcd. for $C_{13}H_{17}N_5O_2 0.55\ H_2O$:
Calcd: C, 54.75; H, 6,40; N, 24.55
Found: C, 54.67; H, 6.43; N; 24.57

Example 4 : 6-n-Propoxypurine-9-β-D-2′,3′-dideoxyribofuranoside

6-n-Propoxypurine (0.5g, 2.8 mmoles, Sigma Chemicals, St. Louis, MO) and 3′-deoxythymidine (0.96g, 4.2 mmoles)(Horwitz, J.P., et al J.Org.Chem., 31, 205 (1996)) were dissolved in 5ml dimethyl sulfoxide and 5ml N,N dimethyl formamide. 30ml of 10mM potassium phosphate buffer, pH 6.8, containing 0.04% potassium azide and purified purine nucleoside phosphorylase (20,000 I.U.) and thymidine phosphorylase (10,000 I.U.) (Krenitsky, T.A., et al., Biochemistry, 20, 3615, 1981 and US Patent 4,381,444) absorbed onto 10ml of DEAE-cellulose resin were added and the reaction was stirred at 35° C for 7 hours. The resin was removed by centrifugation and the supernatant applied to a column of AG1-X2 (OH form), 2.5 x 10cm, coupled to a column of XAD-2, 2.5 x 20cm. The columns were washed with 500ml of water and the product was eluted with methanol. The product was flash chromatographed on a silica gel column, 3 x 50 cm, with chloroform : methanol (9:1 v/v). Lyophilization afforded 6-n-propoxypurine-9-β-D-2′,3′-dideoxyribofuranoside that analyzed as a 0.3 hydrate.
$^1$H NMR (200 MHz, DMSO-$d_6$) δ 8.58 and 8.48 (2s, 2H, $H_2$ and $H_8$), 6.30(dd, 1H, J=5.8 Hz, J=4.3 Hz, $H_1$′), 4.94(t, 1H, J=5.5 Hz, $OH_5$′), 4.48(t, 2H, J=6.7 Hz, $OCH_2CH_2CH_3$), 4.12(m, 1H, $H_4$), 3.54(m, 2H, $H_5$′ and $H_5$″), 2.42(m, 2H, $H_2$′ and $H_2$″), 2.07(m, 2H, $H_3$′ and $H_3$″), 1.80(m, 2H, $OCH_2CH_2CH_3$), 0.98(t, 3H, J=7.4 Hz, $OCH_2CH_2CH_3$).
Analysis Calculated for $C_{13}H_{18}N_4O_3 0.3H_2O$
Calculated : C, 55.04; H, 6.61; N, 19.75
Found : C, 55.05; H, 6.61; N, 19.72

Example 5 : 6-Cyclopropylmethoxypurine-9-β-D-2′,3′-dideoxyribofuranoside

6-Cyclopropylmethoxypurine was prepared by nucleophilic displacement of the chlorine group on 6-chloropurine (Sigma Chemical Co., St. Louis MO) by the alkoxy anion formed between sodium hydride and cyclopropylmethyl alcohol.

6-Cyclopropylmethoxypurine (0.505g, 26.5 mmoles) and 2′,3′-dideoxy- thymidine (0.908g, 40.1 mmoles) (Horwitz et al, J.Org.Chem., 31, 205 (1966)) were reacted and chromatographed on AG1-X2 (OH form) and XAD-2 as described in Example 4. Product containing fractions were flash chromatographed on a silica gel column, 3 x 50 cm, with acetonitrile : water (98:2, v/v). Lyophilization yielded 6-cyclopropylmethoxypurine-9-β-D-2′,3′-dideoxyribofuranoside.

$^1$H NMR (200 MHz, DMSO-$d_6$) δ 8.59 and 8.47 (2s, 2H, $H_2$ and $H_8$), 6.30 (dd, 1H, J=6.0 Hz, J=4.2 Hz, $H_1'$), 4.95 (t, 1H, J=5.5 Hz, 5′-OH), 4.36 (d, 2H, J=7.3 Hz, $OCH_2$), 4.12 (m, 1H, $H_4'$), 3.54 (m, 2H, $H_5'$ and $H_5''$), 2.42 (m, 2H, $H_2'$ and $H_2''$), 2.03 (m, 2H, $H_3'$ and $H_3''$), 1.32 (m, 1H, CH), 0.58 and 0.39 (2 m, 4H, $2CH_2$).

Analysis Calculated for $C_{14}H_{18}N_4O_3$

Calculated : C, 57.92; H, 6.25; N, 19.30

Found : C, 57.99, H, 6.28; N, 19.27

Example 6 : 6-Cyclopentyloxypurine-9-β-D-2′,3′-dideoxyribofuranoside

6-Cyclopentyloxypurine was prepared by nucleophilic displacement of the chlorine group on 6-chloropurine (Sigma Chemical Co., St. Louis, 31, 205 (1966)) by the alkoxy anion formed between sodium hydride and cyclopentanol.

6-Cyclopentyloxypurine (0.506g, 2.48 mmoles) and 3′-deoxythymidine (0.856g, 3.78 mmoles) (Horwitz J.P., et al. J.Org.Chem, 31, 205 (1966)) were reacted and chromatographed on AG1-X2 (OH form) and XAD-2 as described in Example 4. Solvent was removed in vacuo from product fractions and the residue was flash chromatographed on a silica gel column, 3 x 50cm, with chloroform : methanol (95.5, v/v). Lyophilization yielded 6-cyclopentyloxypurine-9-β-D-2′,3′-dideoxyribofuranoside that analyzed as a 0.15 hydrate.

$^1$H NMR (200 MHz, DMSO-$d_6$) δ 8.56 and 8.48 (2s, 2H, $H_2$ and $H_8$), 6.29 (dd, 1H, J=5.8 Hz, J=4.4 Hz, $H_1'$), 5.68 (m, 1H, OCH), 4.95 (t, 1H, J=5.5 Hz, 5′-OH), 4.12 (m, 1H, $H_4'$), 3.56 (m, 2H, $H_5'$ and $H_5''$), 2.42 (m, 2H, $H_2'$ and $H_2''$), 2.03 (b, 4H, $H_3'$, $H_3''$, and $CH_2$), 1.76 (b, 6H, $3CH_2$).

Analysis Calculated for $C_{15}H_{20}N_4O_3$ $0.15H_2O$

Calculated : C, 58.68; H, 6.66: N, 18.25

Found : C, 58.61; H, 6.53, N, 18.25

Example 7 : 2-Amino-6-ethoxypurine-9-β-D-2′,3′-dideoxyribofuranoside

2-Amino-6-ethoxypurine (0.5g, 2.8 mmoles prepared by nucleophilic displacement of the chlorine group on 2-amino-6-chloropurine, (Aldrich Chemical Co., Milwaukee WI) by the alkoxy anion formed between sodium hydride and ethanol) and 3′-deoxythymidine (0.950g, 4.19 mmoles) (Horwitz J.P. et al J.Org.Chem., 31, 205 (1966)) were reacted and chromatographed on AG1-X2 (OH form) and XAD-2 as described in Example 4. Solvent was removed in vacuo from product containing fractions and the residue was flash chromatographed on a silica gel column, 5 x 20 cm, with chloroform : methanol (9:1, v/v). Lyophilization yielded 2-amino- 6-ethoxypurine-9-β-D-2′,3′-dideoxyribofuranoside that analyzed as a 0.3 hydrate (mp 150° C, partial melt at 65° C).

$^1$H NMR (200 MHz, DMSO-$d_6$) : δ 8.07 (s, 1H, $H_8$), 6.35 (b, 2H, $NH_2$), 6.06 (dd, 1H, $H_1'$), 4.92 (t, 1H, J=5.49 Hz, 5′OH), 4.43 (q, 2H J=7.05 Hz, $CH_2$), 4.05 (m 1H, $H_4'$), 3.56 (m, 2H, 5′$CH_2$), 2.32 (m, 2H, 2′$CH_2$), 2.00 (m, 2H, 3′$CH_2$), 1.34 (t, 3H, J=7.08 Hz, $CH_3$).

Analysis Calculated for $C_{12}H_{17}N_5O_3$ $0.3H_2O$

Calculated : C, 50.63; H, 6.23; N, 24.60

Found : C, 50.77; H, 6.21, N, 24.63

Example 8 : 6-sec-Butoxypurine-9-β-D-2′,3′-dideoxyribofuranoside

6-sec-Butoxy-9H-purine was prepared by nucleophilic displacement of the chlorine group on 6-chloropurine (Sigma Chemical Co., St. Louis, MO) by the alkoxy anion formed between sodium hydride and 2-butanol.

6-sec-Butoxy-9H-purine (0.501g, 2.61 mmoles) and 3′-deoxythymidine (0.889g, 3.92 mmoles) (Sigma

Chemical Co., St. Louis, MO) were dissolved in 5ml dimethyl sulfoxide and 5ml N,N-dimethylformamide. Thirty ml of 10mM potassium phosphate buffer, pH 6.8, containing 0.04% potassium azide and purified purine nucleoside phosphorylase (20,000 I.U.) and thymidine phosphorylase (10,000 I.U.) (Krenitsky, T.A., et al., Biochemistry, 20, 3615, 1981 and US Patent 4,381,444) absorbed onto 10ml DEAE-cellulose resin were added and the reaction was stirred at 35 °C for 7 hours. The resin was removed by centrifugation and the supernatant applied to a column of AG1-X2 (OH- form) (2.5 x 10cm) coupled to a column of XAD-2 (2.5 x 20cm). The columns were washed with 500ml water and the product was eluted with methanol. The product was flash chromatographed on a silica gel column, 4.8 x 23cm, with 90% chloroform : 10% methanol (v:v). Appropriate fractions were pooled, solvent was removed in vacuo, and the residue was dissolved in a minimum amount of 80% methanol. This solution was appplied to a $C_{18}$ reverse phase column, 2.5 x 25cm, and eluted with 80% methanol. Lyophilization yielded the title compound.

$^1H$ NMR (200 MHz, DMSO-$d_6$) $\delta$ 8.56 and 8.47 (2s, 2H, $H_2$ and $H_8$), 6.29 (dd, 1H, J=5.7 Hz, J=4.4 Hz, $H_1'$), 5.43 (m, 1H, J=6.2 Hz, $CH(CH_3)CH_2CH_3$), 4.94 (t, 1H, J=5.6 Hz, $OH_5'$), 4.14 (m, 1H, $H_4'$), 3.55 (m, 2H, $H_5'$ and $H_5''$), 2.42 (m, 2H, $H_2'$ and $H_2''$), 2.04 (m, 2H, $H_3'$ and $H_3''$), 1.73 (m, 2H, $CH(CH_3)CH_2CH_3$), 1.33 (d, 3H, J=6.2 Hz, $CH(CH_3)CH_2CH_3$), and 0.92 (t, 3H, J=7.4 Hz, $CH(CH_3)CH_2CH_3$).

Analysis Calculated for $C_{14}H_{20}N_4O_3$

Calculated : C,57.52; H,6.90; N,19,17

Found : C,57.68; H,6.86; N,19.05

Example 9 : 6-[Cyclopropylmethylamino]purine-9-β-D-2′,3′-dideoxyribofuranoside

6-Cyclopropylmethylaminopurine was prepared by nucleophilic displacement of the chlorine group on 6-chloropurine (Sigma Chemicals, St. Louis MO) by the amino group on cyclopropylmethylamine (Sigma Chemicals, St. Louis MO).

6-Cyclopropylmethylaminopurine (2.64 mmol 0.50g) was dissolved in 5 ml of dimethylformamide. After cooling to room temperature 3′-deoxythymidine (3.98 mmol, 0.90 g) (Horwitz, J.P. et al., J. Org. Chem. 31, 205 (1966) was added along with 30 ml of 10 mM potassium phosphate buffer with a pH of 6.8 containing 0.04% potassium azide. Purified thymidine phosphorylase (10,000.I.U.) and purine nucleoside phosphorylase (20,000 I.U.) (Krenitsky T.A. et al., Biochemistry 20, 3615, 1981 and US Patent 4,381,444) absorbed onto 10 ml DEAE cellulose (Whatman) were added, and the suspension was stirred at 30 °C. After 8 hours the reaction was filtered, and the filtrate was applied to a series of coupled columns. The initial column contained AG1-X2 resin (OH-form), 2.5 x 10 cm, while the second column contained Amberlite XAD-2 resin, 2.5 x 20 cm. After sample application, the columns were washed with 500 ml water and the product was eluted with methanol. The product was then flash chromatrographed on a silica gel column, 5 x 20 cm, with a mixture of chloroform:methanol (9:1, v/v). Solvent was removed in vacuo and the product gum was transferred in acetone to a vial. Lyophilisation yielded 0.588 g of 6-cyclopropylmethylaminopurine-9-β-D-2′,3′-dideoxyribofuranoside that analysed for 0.15 water and 0.15 acetone.

$^1H$ NMR (200 MHz, DMSO-$d_6$) $\delta$ 8.34 and 8.25 (2s, 2H, $H_2$ and $H_8$), 6.23 (dd, 1H, J=4.29, J=4.30, $H_1'$), 4.10 (m, 1H, $H_4'$), 3.59 and 3.53 (m, 2H, $H_5'$ and $H_5''$), 3.36 (s, 3H, N-$CH_3$), 3.28 (b, 1H, cyclopropyl CH), 2.4 (b, 2H, $H_2'$ and $H_2''$), 2.07 (m, 2H, $H_3'$ and $H_3''$), 0.88 and 0.72 (2m, 4H, cyclopropyl $CH_2CH_2$).

Analysis Calculated for $C_{14}H_{19}N_5O_2$ 0.15 $H_2O$ 0.15 $C_3H_6O$:

Calculated : C, 57.71; H, 6.77; N, 23.29

Found : C, 57.73; H, 6.94; N, 23.39

The following illustrate pharmaceutical formulations according to the invention wherein the "Active ingredient" comprises components (a) and (b) above in a ratio of 1:4, component (b) advantageously being 6-[cyclopropyl methylamino]purine-9-β-D-2′,3′-dideoxyribofuranoside.

Example A : Tablet Formulations

The following formulations A, B and C are prepared by wet granulation of the ingredients with a solution of povidone, followed by addition of magnesium stearate and compression.

| Formulation A | | |
|---|---|---|
| | mg/tablet | mg/tablet |
| (a) Active ingredient | 250 | 250 |
| (b) Lactose B.P. | 210 | 26 |
| (c) Povidone B.P. | 15 | 9 |
| (d) Sodium Starch Glycollate | 20 | 12 |
| (e) Magnesium Stearate | 5 | 3 |
| | 500 | 300 |

| Formulation B | | |
|---|---|---|
| | mg/tablet | mg/tablet |
| (a) Active ingredient | 250 | 250 |
| (b) Lactose | 150 | - |
| (c) Avicel PH 101 | 60 | 26 |
| (d) Povidone B.P. | 15 | 9 |
| (e) Sodium Starch Glycollate | 20 | 12 |
| (f) Magnesium Stearate | 5 | 3 |
| | 500 | 300 |

| Formulation C | |
|---|---|
| | mg/tablet |
| Active ingredient | 100 |
| Lactose | 200 |
| Starch | 50 |
| Povidone | 5 |
| Magnesium stearate | 4 |
| | 359 |

The following formulations, D and E, are prepared by direct compression of the admixed ingredients. The lactose in formulation E is of the direct compression type (Dairy Crest - "Zeparox").

| Formulation D | |
|---|---|
| | mg/tablet |
| Active ingredient | 250 |
| Pregelatinised Starch NF15 | 150 |
| | 400 |

| Formulation E | |
|---|---|
| | mg/tablet |
| Active ingredient | 250 |
| Lactose | 150 |
| Avicel | 100 |
| | 500 |

Formulation F (Controlled Release Formulation)

The formulation is prepared by wet granulation of the ingredients (below) with a solution of povidone followed by the addition of magnesium stearate and compression.

| | mg/tablet |
|---|---|
| (a) Active ingredient | 500 |
| (b) Hydroxypropylmethylcellulose (Methocel K4M Premium) | 112 |
| (c) Lactose B.P. | 53 |
| (d) Povidone B.P. | 28 |
| (e) Magnesium Stearate | 7 |
| | 700 |

Drug release takes place over a period of about 6-8 hours and is complete after 12 hours.

Example B : Capsule Formulations

Formulation A

A capsule formulation is prepared by admixing the ingredients of Formulation D in Example A above and filling into a two-part hard gelatin capsule. Formulation B (infra) is prepared in a similar manner.

| Formulation B | |
|---|---|
| | mg/capsule |
| (a) Active ingredient | 250 |
| (b) Lactose B.P. | 143 |
| (c) Sodium Starch Glycollate | 25 |
| (d) Magnesium Stearate | 2 |
| | 420 |

| Formulation C | |
|---|---|
| | mg/capsule |
| (a) Active ingredient | 250 |
| (b) Macrogol 4000 B.P. | 350 |
| | 600 |

Capsules of formulation C are prepared by melting the Macrogol 4000 BP, dispersing the active ingredient in the melt and filling the melt into a two-part hard gelatin capsule.

| Formulation D | |
|---|---|
| | mg/capsule |
| Active ingredient | 250 |
| Lecithin | 100 |
| Arachis Oil | 100 |
| | 450 |

Capsules of formulation D are prepared by dispersing the active ingredient in the lecithin and arachis oil and filling the dispersion into soft, elastic gelatin capsules.

Formulation E (Controlled Release Capsule)

The following controlled release capsule formulation is prepared by extruding ingredients (a), (b) and (c) below using an extruder, followed by spheronisation of the extrudate and drying. The dried pellets are then coated with release- controlling membrane (d) and filled into a two-piece, hard gelatin capsule.

| | mg/capsule |
|---|---|
| (a) Active ingredient | 250 |
| (b) Microcrystalline Cellulose | 125 |
| (c) Lactose B.P. | 125 |
| (d) Ethyl Cellulose | 13 |
| | 513 |

| Example C : Injectable Formulation | |
|---|---|
| Formulation A | |
| Active ingredient | 0.200g |
| Hydrochloric acid solution, 0.1M, or Sodium hydroxide solution, 0.1M q.s. to pH | 4.0 to 7.0 |
| Sterile water q.s. to | 10ml |

The active ingredient is dissolved in most of the water (35°-40°C) and the pH adjusted to between 4.0 and 7.0 with the hydrochloric acid or the sodium hydroxide as appropriate. The batch is then made up to volume with the water and filtered through a sterile micropore filter into a sterile 10ml amber glass vial (type 1) and sealed with sterile closures and overseals.

| Formulation B | |
|---|---|
| Active ingredient | 0.125 g |
| Sterile, pyrogen-free, pH 7 phosphate buffer, q.s. to | 25 ml |

| Example D : Intramuscular Injection | |
|---|---|
| Active ingredient | 0.20 g |
| Benzyl Alcohol | 0.10 g |
| Glycofurol 75 | 1.45 g |
| Water for Injection q.s. to | 3.00 ml |

The active ingredient is dissolved in the glycofurol. The benzyl alcohol is then added and dissolved, and water added to 3 ml. The mixture is then filtered through a sterile micropore filter and sealed in sterile 3 ml amber glass vials (type 1).

| Example E : Syrup | |
|---|---|
| Active ingredient | 0.25 g |
| Sorbitol Solution | 1.50 g |
| Glycerol | 2.00 g |
| Sodium Benzoate | 0.005 g |
| Flavour, Peach 17.42.3169 | 0.0125 ml |
| Purified Water q.s. to | 5.00 ml |

The active ingredient is dissolved in a mixture of the glycerol and most of the purified water. An aqueous solution of the sodium benzoate is then added to the solution, followed by addition of the sorbitol solution and finally the flavour. The volume is made up with purified water and mixed well.

| Example F : Suppository | |
|---|---|
| | mg/suppository |
| Active ingredient (63μm)* | 250 |
| Hard Fat, BP (Witepsol H15 - Dynamit NoBel) | 1770 |
| | 2020 |

*The active ingredient is used as a powder wherein at least 90% of the particles are of 63μm diameter or less.

One-fifth of the Witepsol H15 is melted in a steam-jacketed pan at 45°C maximum. The active ingredient is sifted through a 200μm sieve and added to the molten base with mixing, using a silverson fitted with a cutting head, until a smooth dispersion is achieved. Maintaining the mixture at 45°C, the remaining Witepsol H15 is added to the suspension and stirred to ensure a homogenous mix. The entire suspension is passed through a 250μm stainless steel screen and, with continuous stirring, is allowed to cool to 40°C. At a temperature of 38°C to 40°C, 2.02g of the mixture is filled into suitable, 2 ml plastic moulds. The suppositories are allowed to cool to room temperature.

| Example G : Topical formulation | |
|---|---|
| | Cream |
| Active compound | 5.00g |
| Glycerol | 2.00g |
| Cetostearyl alcohol | 6.75g |
| Sodium lauryl sulphate | 0.75g |
| White soft paraffin | 12.50g |
| Liquid paraffin | 5.00g |
| Chlorocresol | 0.01g |
| Purified water to | 100.00g |

The active compound is dissolved in a mixture of purified water and glycerol and heated to 70° C. The remaining ingredients are heated together at 70° C. The two parts are added together and emulsified, cooled and filled into containers.

| Example H : Pessaries | |
|---|---|
| | mg/pessary |
| Active ingredient (63µm) | 250 |
| Anhydrate Dextrose | 380 |
| Potato Starch | 363 |
| Magnesium Stearate | 7 |
| | 1000 |

The above ingredients are mixed directly and pessaries prepared by direct compression of the resulting mixture.

Antiviral Activity

Purine compounds according to the invention and zidovudine were tested for anti-HIV activity in an HIV-infected MT4 cell assay (Averett, D.R., J. Virol. Methods 23, 263-276, (1989)). The cells were exposed to HIV for one hour prior to addition of antiviral compound(s). Compounds were tested in serial 2.5-fold dilutions. After 5 days of incubation at 37° C, the cell number was determined. Inhibition of HIV-induced cytopathic effect was calculated, and synergism was determined by FIC plots as described by Elion, Singer and Hutchings, J.Biol.Chem., 208, 477 (1954).

A fractional inhibitor concentration (FIC) index of less than 1 indicates synergy while a FIC index of 1 indicates additivity.

In these plots, the concentrations of antiviral agents required to manifest 50% inhibition of the virus are determined from the experimental data for each agent separately and for the combination of two agents at a variety of ratios. The amount of each drug which is present in the combination is then compared to the amount of each drug which would be required to manifest such an effect if the agents acted in a strictly additive fashion. This comparison provides the fractional inhibitory concentration for each agent of the combination. The fractional inhibitory concentrations for each of the two agents in the combination are then summed, to provide the FIC index. The degree to which this FIC index is less than 1 is a measure of the degree of synergy. Since certain ratios of each agent may provide more or less synergy in the present invention, the results are shown for five different concentrations of zidovudine.

| FIC Index at Three Zidovudine Concentrations | | | | | |
|---|---|---|---|---|---|
| EXAMPLE | 33nm | 25 nM | 12.5 nM | 11nm | 6.25nM |
| 1 | | 0.82 | 0.77 | | 0.76 |
| 2 | | 0.77 | 0.76 | | 0.96 |
| 3 | | 0.60 | 0.64 | | 0.86 |
| 4 | | 0.69 | 0.73 | | 0.62 |
| 5 | | 0.88 | 0.77 | | 0.79 |
| 6 | | 0.80 | 0.63 | | 0.91 |
| 7 | | 0.92 | 0.78 | | 0.60 |
| 8 | | 0.68 | 0.46 | | 0.54 |
| 9 | 0.80 | | | 0.66 | |

## Claims

1. A combination of:
(a) zidovudine and
(b) at least one 2′,3′-dideoxypurine nucleoside selected from;
i) 6-Ethylaminopurine-9-β-D-2′,3′-dideoxyribofuranoside;
ii) 6-Dimethylaminopurine-9-β-D-2′,3′-dideoxyribofuranoside;
iii) 6-Cyclopropylaminopurine-9-β-D-2′,3′-dideoxyribofuranoside;
iv) 6-n-Propoxypurine-9-β-D-2′,3′-dideoxyribofuranoside;
v) 6-Cyclopropylmethoxypurine-9-β-D-2′,3′-dideoxyribofuranoside;
vi) 6-Cyclopentyloxypurine-9-β-D-2′,3′-dideoxyribofuranoside;
vii) 2-Amino-6-ethoxypurine-9-β-D-2′,3′-dideoxyribofuranoside;
viii) 6-sec-Butoxypurine-9-β-D-2′,3′-dideoxyribofuranoside; and
ix) 6-Cyclopropylmethylaminopurine-9-β-D-2′,3′-dideoxyribofuranoside
or a pharmaceutically acceptable derivative thereof, components (a) and (b) of the combination being employed together such that a potentiated antiviral effect is achieved.

2. A combination as claimed in claim 1 wherein component (b) is 6-cyclopropylaminopurine-9-β-D-2′,3′-dideoxyribofuranoside.

3. A combination as claimed in claim 1 wherein component (b) is 6-cyclopropylmethylamino purine-9-β-D-2′,3′-dideoxyribofuranoside.

4. A pharmaceutical formulation comprising a combination as claimed in any of claims 1 to 3 together with at least one pharmaceutical carrier.

5. A pharmaceutical formulation as claimed in claim 4 in the form of a tablet or capsule.

6. A combination as claimed in any of claims 1 to 3 for use in medical therapy.

7. A combination as claimed in claim 6 for use in the treatment or prophylaxis of viral infections.

8. A combination as claimed in claim 7 for use in the treatment or prophylaxis of human immunodeficiency virus infections.

9. A combination as claimed in claim 6 for use in the treatment or prophylaxis of acquired immune deficiency syndrome.

10. Use of a combination as claimed in any of claims 1 to 3 in the manufacture of a medicament for the treatment or prophylaxis of human immunodeficiency virus infections.

11. A combination as claimed in claim 6 for use in the treatment or prophylaxis of hepatitis B virus infections.

European Patent Office

# EUROPEAN SEARCH REPORT

Application Number

EP 89 31 0146

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A | THE LANCET, vol. II, no. 8573, 19th December 1987, pages 1469-70, London, GB: T. RUZICKA: "Treatment of HIV-induced retinoid-restistant psoriasis with zidovudine" * Page 1469, column 2 * --- | 1 | A 61 K 31/70 |
| P,X | EP-A-0 327 200 (THE UNIVERSITY OF LIVERPOOL) * Claims 1-4; page 4, lines 47-50 * ----- | 1 | |
| | | | TECHNICAL FIELDS SEARCHED (Int. Cl.5) |
| | | | A 61 K |

The present search report has been drawn up for all claims

| Place of search | Date of completion of the search | Examiner |
|---|---|---|
| THE HAGUE | 24-01-1990 | PEETERS J.C. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)